# EUROPEAN PATENT APPLICATION

(11) **EP 4 755 177 A1**
(43) Date of publication of application: **10.06.2026**
(21) Application number: 25176503.8
(22) Date of filing: 14.05.2025
(51) Int. Cl.: A01K 1/00, A01K 29/00

(54) **ANIMAL SELECTION AND BREEDING METHOD, ELECTRONIC DEVICE, COMPUTER-READABLE STORAGE MEDIUM, AND COMPUTER PROGRAM PRODUCT**

(30) Priority: 09.12.2024 CN 202411795348
(71) Applicant: Beijing Etag Technology Company., Ltd., Haidian District Beijing 100083 (CN)
(72) Inventor: ZHOU, Xingfu, Beijing, 100083 (CN); REN, Jiping, Beijing, 100083 (CN); PANG, Chao, Beijing, 100083 (CN); HU, Dongge, Beijing, 100083 (CN); DONG, Jiemin, Beijing, 100083 (CN); DING, Shijie, Beijing, 100083 (CN); LI, Jingkuo, Beijing, 100083 (CN); ZHOU, Jingyi, Beijing, 100083 (CN)
(74) Representative: Dilg, Haeusler, Schindelmann Patentanwaltsgesellschaft mbH

(57) **Abstract**

The embodiments of the present disclosure provide an animal selection and breeding method, an electronic device, a computer-readable storage medium, and a computer program product. Each animal wears the communication transmitting device, and the corresponding communication receiving device is mounted in the activity area, which realizes real-time monitoring of the physiological data for the animal growth process. Later, the backup animals meeting the selection requirements are determined based on the physiological data, and the backup animals are automatically divided into the breeding area through the dividing device, which improves the accuracy of selection and breeding, and realizes the automatic management of the selection and breeding process.

## Description

### TECHNICAL FIELD

The present disclosure relates to the technical field of animal genetic breeding, and specifically to an animal selection and breeding method, an electronic device, a computer-readable storage medium, and a computer program product.

### BACKGROUND ART

The animal selection and breeding are mainly aimed at young economic animals, wherein the main purpose of selection and breeding is to obtain backup animals with good development, robust physique, distinctive breed characteristics, and high breeding value for breeding. Through selection and breeding, it can ensure that the economic animal groups have excellent production performance, such as high fertility, good disease resistance, and high-quality meat, which is conducive to maintaining the production level and economic efficiency of economic animal breeding farms.

However, in the related art, the ways and methods of selecting and breeding the backup animals are not diversified, and it fails to select the backup animals meeting comprehensive requirements, so the selection and breeding of backup animals have errors and are not precise enough, thereby affecting the production level of the breeding farm.

### SUMMARY

The objective of embodiments of the present disclosure is to provide an animal selection and breeding method, an electronic device, a computer-readable storage medium, and a computer program product for realizing the technical effect of improving the accuracy of animal selection and breeding.

A first aspect of the embodiments of the present disclosure provides animal selection and breeding method, wherein each animal wears a first communication transmitting device, and a dividing device and a first feeding device for feeding the animal are mounted in an activity area of animals, wherein a first communication receiving device is mounted on the first feeding device, and the first communication receiving device is communicatively connected to the first communication transmitting device in a preset range; and the method includes:
obtaining physiological data of each animal at one or more growth stages when receiving a breeding instruction, wherein the growth stages are divided based on ages of the animals, and at least a part of the physiological data is determined based on communication interaction data between the first communication transmitting device and the first communication receiving device;
determining backup animals meeting preset selection requirements from the multiple animals based on the physiological data; and
sending a dividing instruction with an identification of the backup animals to the dividing device, wherein the dividing instruction is configured to instruct the dividing device to divide the backup animals to a breeding area.

In the above realization process, each animal wears the communication transmitting device, and the corresponding communication receiving device is mounted in the activity area, which realizes real-time monitoring of the physiological data for the animal growth process. Then, the backup animals meeting the selection requirements are determined based on the physiological data, and the backup animals are automatically divided into the breeding area through the dividing device, which improves the accuracy of selection and breeding, and realizes the automatic management of the selection and breeding process.

Further, the growth stages of the animal include a first growth stage and a second growth stage experienced sequentially; the animals are fed different foods during the first growth stage and the second growth stage; and the physiological data of the second growth stage includes any one or more data of animal eating data, animal activity data, animal weight data, first environmental suitability data of the activity area, animal health condition data, and animal inbreeding condition data, wherein the animal eating data and the animal activity data are determined based on the communication interaction data between the first communication transmitting device and the first communication receiving device; the animal weight data is determined based on a first weight estimation device integrated into the first feeding device; and the first environmental suitability data is used to characterize a degree of environmental suitability of the animals.

In the above realization process, the growth stages of the animal are divided in detail, and the physiological data of each stage is collected in a targeted manner. In the second growth stage, various physiological data including the animal eating data, activity data, weight data, environmental suitability data, health condition data, and inbreeding condition data are comprehensively collected. These data provide an important basis for accurately assessing the growth condition, breeding potential, and health condition of the animals, and it helps to improve the accuracy of selection and breeding when selecting and breeding backup animals by using these data.

Further, the growth stages of the animal include a first growth stage and a second growth stage experienced sequentially; the animals are fed different foods during the first growth stage and the second growth stage; the physiological data of the first growth stage includes any one or more data of animal activity data, animal birth weight data, animal weaning weight data, second environmental suitability data of the activity area, animal mother eating data, animal mother health condition data, animal health condition data, and animal inbreeding condition data, wherein the animal activity data is determined based on the communication interaction data between the first communication transmitting device and the first communication receiving device, and the second environmental suitability data is used to characterize a degree of environmental suitability of the animal mother.

In the above realization process, for the first growth stage, various physiological data including animal activity, birth weight, weaning weight, environmental suitability of the activity area, data of the eating and health condition of the animal mother, animal health condition data, and inbreeding condition data are collected. These data not only comprehensively reflect the growth condition and selective breeding potential of the animals, but also fully consider the influence of mother factors on the animals. Through analyzing these data, it is more beneficial to select and breed the backup animals with distinctive breed characteristics and high breeding values.

Further, the activity area includes a second weight estimation device, and the animal birth weight data and the animal weaning weight data are determined by the second weight estimation device.

In the above realization, the second weight estimation device is introduced, which realizes the precise collection of critical weight data during the selection and breeding process of the backup animals.

Further, the animal mother wears a second communication transmitting device, and a second feeding device for feeding the animal mother is further mounted in the activity area, wherein the animal mother eating data is determined based on communication interaction data between the second communication transmitting device and a communication receiving device mounted on a second feeding device.

In the above realization, the animal mother is wearing the communication transmitting device, and the communication receiving device communicatively connected to the communication transmitting device is mounted on the feeding device for feeding the animal mother, which realizes the collection of animal mother eating data.

Further, an environmental detection device is integrated into each feeding device in the activity area, and the environmental detection device is configured to determine the environmental suitability data.

In the above realization process, the environmental suitability data can be determined by the environmental detection device integrated into the feeding device, and the tolerance degree of the animal or the animal mother to the harsh environment can be analyzed based on the data, so as to determine the physical performance of the animal.

Further, the step of determining backup animals meeting preset selection requirements from the multiple animals based on the physiological data includes:
for each of the growth stages, determining a first breeding value of each of the growth stages according to each piece of the physiological data in the growth stages and a weight coefficient respectively corresponding to each piece of the physiological data;
calculating a second breeding value according to the first breeding value of each of the growth stages and a weight coefficient corresponding to each of the growth stages; and
determining the backup animal according to the second breeding value of each animal.

In the above realization process, reasonable values are assigned to the physiological data of different growth stages by introducing the weight coefficient, which ensures the comprehensiveness and accuracy of the selection and breeding process. Meanwhile, the first breeding value is weighted according to the degree of influence of different growth stages on the selection and breeding, which further improves the science and accuracy of the selection and breeding.

A second aspect of the embodiments of the present disclosure provides an electronic device, wherein the electronic device includes:
a processor,
configured to store a memory of processor-executable instructions, wherein
any one of the methods described in the first aspect is realized when the processor invokes the executable instructions.

A third aspect of the embodiments of the present disclosure provides a computer-readable storage medium with computer instructions stored thereon, wherein steps in any one of the methods described in the first aspect are realized when the computer instructions are executed by the processor.

A fourth aspect of the embodiments of the present disclosure provides a computer program product, wherein the computer program product includes a computer program, and any one of the methods in the first aspect is realized when the computer program is executed by the processor.

### BRIEF DESCRIPTION OF DRAWINGS

In order to more clearly illustrate the technical solutions in the embodiments of the present disclosure, the drawings to be used in the embodiments of the present disclosure will be briefly introduced below. For persons of ordinary skill in the field, other relevant drawings can be obtained according to these drawings without inventive efforts. For persons of ordinary skill in the field, other relevant drawings can be obtained according to these drawings without inventive efforts.
FIG. 1 shows a schematic flow diagram of an animal selection and breeding method provided by the embodiments of the present disclosure;
FIG. 2 shows a structural schematic diagram of a dividing device provided by the embodiments of the present disclosure;
FIG. 3 shows a schematic diagram of a communication transmitting device provided by the embodiments of the present disclosure;
FIG. 4 shows a schematic diagram of a positional relationship between a communication receiving device and a feeding device provided by the embodiments of the present disclosure;
FIG. 5 shows a schematic diagram of different types of feeding devices provided by the embodiments of the present disclosure; and
FIG. 6 shows a structure schematic diagram of an electronic device provided by the embodiments of the present disclosure.

### DETAILED DESCRIPTION OF EMBODIMENTS

The technical solutions in the embodiments of the present disclosure will be described below in conjunction with the drawings in the embodiments of the present disclosure.

It should be noted that similar symbols and letters denote similar items in the following drawings, so that once an item is defined in a drawing, no further definition or explanation is required in the subsequent drawings. At the same time, in the description of the present disclosure, the terms, "first", "second", etc., are used only to differentiate the description, and are not to be understood as indicating or implying relative importance.

In related art, it is usually preferred to select individuals with larger litter sizes, no genetic diseases, physical appearance meeting breed standards, and fast growth rate from the original population at parity 2 to 5 as backup animals for population replenishment. However, this process is highly dependent on genetic hereditary information and lineage records, and the measurement of the animal phenotypic data is often limited to a particular growth stage of the animal. At present, a full-chain phenotypic data system, which is closely related to selection and breeding and covers identification, behavioral characteristics, weight changes, feeding habits, disease records, and environmental factors, has not been established. The fragmented data collection, reliance on manual records, and poor storage management lead to data disorganization, which in turn leads to the increase of the inbreeding risk for the backup animal during the selection and breeding process and causes generational degeneration. It causes a serious threat to the sustained and stable production and the long-term economic performance of breeding farm, which results in economic losses. Therefore, there is an urgent need for a better system of data collection, management, and analysis, so as to support more accurate and efficient selection and breeding for backup animals.

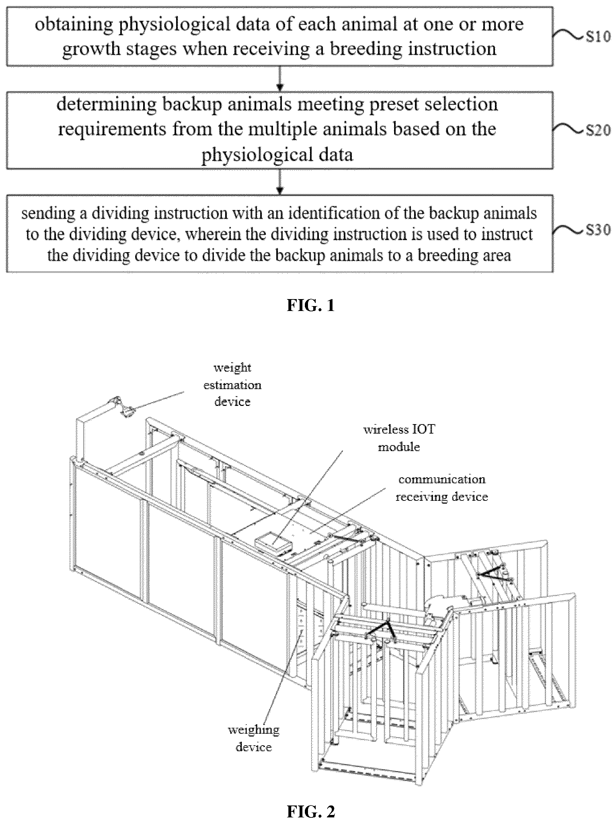

In response to any one issue raised above, the embodiments of the present disclosure provide an animal selection and breeding method. Referring to FIG. 1, FIG. 1 shows a schematic flow diagram of an animal selection and breeding method provided by the embodiments of the present disclosure.

In the embodiment, each animal wears a first communication transmitting device, and a dividing device and a first feeding device for feeding the animal are mounted in an activity area of animals, wherein a first communication receiving device is mounted on the first feeding device, and the first communication receiving device is communicatively connected to the first communication transmitting device in a preset range. The method includes the following steps: S10~S30.

Step S10: obtaining physiological data of each animal at one or more growth stages when receiving a breeding instruction, wherein the growth stages are divided based on ages of the animals, and at least a part of the physiological data is determined based on communication interaction data between the first communication transmitting device and the first communication receiving device.

It should be noted that the execution main body of the embodiment can be a cloud server or any one device described in the embodiment, such as a communication transmitting device, a communication receiving device, a feeding device, and a dividing device. The embodiment takes the execution main body as a cloud server for example.

The animal selection and breeding are mainly aimed at young economic animals. Exemplarily, the animals are pigs. The animal selection and breeding method applied to pigs is used to select backup pigs with good development, robust physique, distinctive breed characteristics, and high breeding value for breeding.

In the activity area of the animals, a dividing device and a first feeding device are mounted. The dividing device is configured to separate animals into different areas according to demands. As shown in FIG. 2, FIG. 2 shows a structure schematic diagram of a dividing device provided by the embodiments of the present disclosure. The dividing device is located at the middle aisle of the activity area, the animal activity area can also be the activity area of the animal mother, and the activity area of the animal and the animal mother can be the same. As an example, the dividing device separates the activity area into various sub-areas during the different growth stages of the animal. During the growth-finishing stage of the animals, the dividing device separates the animal activity area into a drinking area, a feeding area, and a transition area. The dividing device is the hub of the three areas and connects the three areas by the entrance and the exit. The animals in the drinking area can only unidirectionally enter the other two areas (where only one exit door of one area can be opened at the same time according to the different needs of feeding and breeding) via the dividing device (of which only a single animal can pass through each time). The animal entering the transition area can only turn out and cannot go back to the other two areas. The animal in the feeding area can return to the drinking area to drink and lie down through a special one-way channel. After the animal enters the growth-finishing stage, the feeding and the data uploading are accomplished on demand by a group feeding device mounted in the feeding area; the communication receiving device (integrated into the group feeding device) is used to collect the identity, the visiting frequency, and the duration data, so as to evaluate the animal activity and feeding quantity; and the environmental detection device (integrated into the group feeding device) is used to collect and upload the macro-environmental data. The weight estimation device shown in the figure can be an electronic scale, and the weight estimation device can be a camera. The animal image is captured by the camera, and the image is analyzed by using the computer vision technology, so as to estimate the weight of the animal. During the practical use, the weight data of the animal or the animal mother can be collected by a weighing device in the dividing device or a weight estimation device in the dividing device, or both devices can be utilized for the collection of the weight data.

The breeding instructions can be inputted by the manager of the breeding farm and usually carry the number of backup animals to be bred.

The growth stages are divided based on the ages of the animal, and different growth stages correspond to different physiological characteristics and nutritional needs of the animal. Exemplarily, the growth stage of the animal can include a farrowing stage (i.e., the newborn stage, the period from birth in the farrowing until being transferred to the nursery, and the farrowing stage generally last a few days), a nursery stage (i.e., the period from weaning to 70 days of age, and the nursery stage generally lasts 40 days), and a growing-finishing stage (i.e., the period after the nursery stage and before the day of release, and the growing-finishing stage generally lasts 6 months); and the growth stage of the animal can also be divided into a nursery stage (i.e., the period of 1-2 months after the animal is born), and a fattening stage (i.e., the period after the nursery stage and before the day of release). The embodiments do not limit the division of growth stages.

The physiological data includes, but is not limited to weight, activity level, and health condition. The physiological data is used to assess the breeding potential of the animal.

Each animal wears a communication transmitting device from its birth. When the animal wearing the communication transmitting device enters the communication radiation range of the communication receiving device during its activity, the communication receiving device will be communicatively connected to the communication transmitting device. Therefore, the animal behavior can be monitored based on communication interaction data between the communication transmitting device and the communication receiving device. The communication transmitting device and the communication receiving device are configured to record the animal identity information, gender information, location information, behavioral frequency and behavioral duration information, and animal temperature information (where the temperature sensor is integrated into the communication transmitting device, and the communication transmitting device is worn on the animal, so that the animal temperature can be collected by the temperature sensor).

### Communication transmitting device

Specific configuration: the communication transmitting device can be a small and lightweight device, so that it can be easily worn on the animal without interfering with its normal activities. Exemplarily, the communication transmitting device can be worn on the ear of the animal. The communication transmitting device can also be an embedded sensor. The embodiment does not limit the specific form of the communication transmitting device.

Technology realization: as an example, the sensor, processor, and wireless communication module are integrated inside the communication transmitting device.

### Communication receiving device

Specific configuration: the communication receiving device is mounted on the feeding device of the animal. The communication receiving device can be wall-mounted, column-mounted, or embedded, so as to facilitate receiving data from the communication transmitting device. Additionally, the communication receiving device can be mounted at other locations, e.g., any one position in the eating area.

Technology realization: as an example, the receiving device transmits the physiological data of the animal to the server via a UHF (ultra-high frequency) antenna module that is also mounted on the feeding device.

The wireless communication technology utilized between the communication transmitting device and the communication receiving device can be one or more of the following: Bluetooth, Wi-Fi, Zigbee, LoRa, NB-IoT, etc.

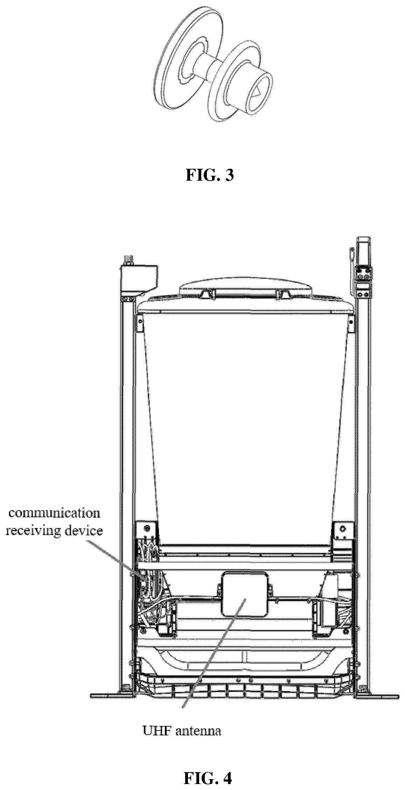

As an example, the communication transmitting device is an ultra-high frequency RFID tag, i.e., a micro-energy sensing tag; the communication receiving device is an ultra-high frequency RFID reader, and the ultra-high frequency RFID reader adopts an ARM Cortex-M3 processor, wherein the operating frequency is 72MHz, the capacity of a built-in Flash memory is 512KB; and a RAM capacity is 64KB; and the operating frequency of an ultra-high frequency antenna module is 915MHz, and the communication distance is controlled to be within 0.5 meters of the near field. Based on ultra-high frequency RFID technology, by using the ultra-high frequency RFID reader and the corresponding micro-energy sensing tag, the near-field contactless identification and information reading for the animal can be realized, so as to obtain the animal behavior data and other related data. As shown in FIG. 3, FIG. 3 is a schematic diagram of a communication transmitting device provided by the embodiment of the present disclosure, wherein the communication transmitting device shown in the figure is worn on the ear of the animal. As shown in FIG. 4, FIG. 4 shows a schematic diagram of the positional relationship between the communication receiving device and the feeding device provided by the embodiment of the present disclosure. The structure shown in the figure is the feeding device, and the communication receiving device and the ultra-high frequency antenna are attached to the feeding device.

Step S20: determining backup animals meeting preset selection requirements from the multiple animals based on the physiological data.

It is to be understood that the preset selection requirements can be numerical ranges of the characterization data. Within this numerical range, the animal is considered to be well-developed, physically robust, possessing distinctive breed characteristics and having high breeding value. The preset selection requirements can be reasonably set according to the breeding goal and actual situation of the breeding farm, and the present embodiments do not limit this.

Step S30: sending a dividing instruction with an identification of the backup animals to the dividing device, wherein the dividing instruction is used to instruct the dividing device to divide the backup animals to a breeding area.

It is to be noted that the breeding area can be an area actually divided from the animal activity area for especially breeding the backup animal, or it can be an existing area (such as the feeding area or the drinking area) temporarily designated as a breeding area during the selection and breeding process, wherein the breeding area serves as a transit station to the area where the backup animals are actually bred and cultivated. After entering the transit station, the backup animals are uniformly driven to the area where the backup animals are actually bred and cultivated. After completing the selection and breeding, the transit station restores its initial regional function (such as returning to the feeding function or the drinking function).

Specifically, one or multiple animals live in one activity area; one breeding farm can have multiple activity areas; and the dividing device and the feeding device are placed in each of the activity areas. For daily eating and drinking, each animal needs to go to the corresponding feeding area or drinking area separated by the dividing device via the dividing device, and the animal can only unidirectionally enter a sub-area at a time after entering the dividing device. Of course, the dividing device can also separate the activity area into more sub-areas, such as the feeding area, the drinking area, and the transition area. An identification module is integrated into the dividing device. When an animal passes through the dividing device, the identification of the animal such as an ID number, can be recognized. When the dividing device receives the identification of the selected backup animal, the backup animal enters the dividing device, and the dividing device determines that it is an animal required to be divided into the breeding area and immediately opens the exit door to the breeding area, so that the animal can only leave the dividing device through the exit door to the breeding area, thereby ultimately completing the division of the backup animal.

In the embodiment, the collection of physiological data of the animal is realized by introducing the communication transmitting device and the communication receiving device; at the same time, the breeding potential of each animal is evaluated based on the physiological data, so as to select more qualified backup animals; and finally, the selected backup animals are divided into the breeding area by the dividing device, so as to complete the precise and high efficient selection and breeding for the backup animals.

Based on any one of the embodiments described above, the growth stages of the animal include a first growth stage and a second growth stage experienced sequentially. The animals are fed different foods during the first growth stage and the second growth stage, and the physiological data of the second growth stage includes any one or more the following data: animal eating data, animal activity data, animal weight data, first environmental suitability data of the activity area, animal health condition data, and animal inbreeding condition data, wherein the animal eating data and the animal activity data are determined based on the communication interaction data between the first communication transmitting device and the first communication receiving device; the animal weight data is determined based on a first weight estimation device integrated into the first feeding device; and the first environmental suitability data is used to characterize a degree of environmental suitability of the animals.

It is to be noted that the first growth stage can refer to an initial stage of the animal growth, including a lactation period and an early suitability period after weaning, in which the animal mainly relies on maternal milk or undergoes a gradual transition to a specific weaning feed.

The second growth stage can refer to a subsequent stage of the animal growth, which occurs after the first growth stage. During this stage, the food of the animal changes from maternal milk or weaning feed to a more complex feed formulation, so as to meet the needs of the rapid growth and development. Of course, during the practical use, the growth stage of the animal can be not limited to these two stages. The person skilled in the art can further divide the first growth stage into multiple substages and/or further divide the second growth stage into multiple substages based on the practical needs. Taking selection and breeding animals as pigs as examples, the first growth stage of pigs can be divided into the farrowing stage (during which the pig is born, and the stage lasts for a few hours or days) and the lactation stage (the period after the farrowing stage and before the weaning day), and the second growth stage of the pig can be further divided into multiple substages, e.g. the second growth stage can be further divided into the nursery stage (the stage during which pigs are weaned to the end of the nursery, lasting from 3 to 5 weeks) and the growing-finishing stage (the stage when the nursery is finished and the pigs enter the growth house until the pigs are released).

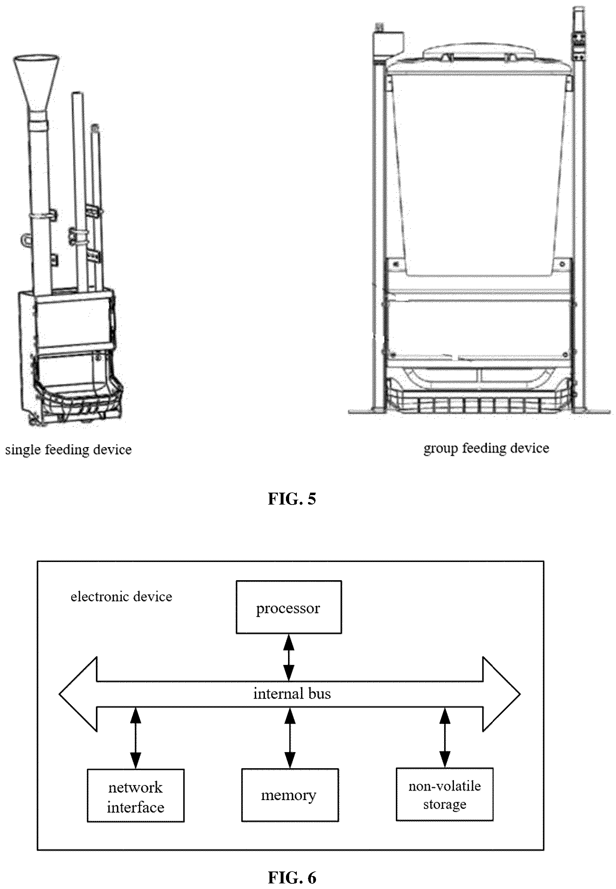

Animal eating data includes the amount of food eaten by the animal during the second growth stage. Specifically, in the embodiment and the following embodiments, the feeding device consists of two parts: various types of sensors and a feeding mechanism, wherein the sensors mainly detect the material level and the eating desire of the animal, and the feeding mechanism mainly accomplishes the feeding on demand the calculation of the feeding amount. The accurate detection of the sensor minimizes the amount of waste, so that the feeding amount is infinitely close to the eating amount. This is because before feeding, the sensor will detect whether there is any feed left and how much feed is left, and then the feeding mechanism calculates the feeding amount based on the amount of feed left detected by the sensor, so that the single feeding amount can be infinitely close to the eating amount. The feeding devices are divided into the single feeding device for feeding a single animal and the group feeding device for feeding a plurality of animals. The daily feeding amount of the single feeding device is equal to the daily eating amount of an animal. For the group feeding device, when the feeding amount is determined, the daily eating amount of an animal is calculated based on the proportional relationship between the total daily eating duration of the animal individual and the total daily eating duration of the animal group. Whether the feeding device for the animal is a single feeding device or a group feeding device, the animal eating data can be calculated from the feeding amount and the eating duration, wherein the eating duration is determined based on the interaction duration between the communication transmitting device and the communication receiving device. As shown in FIG. 5, FIG. 5 shows a schematic diagram of different types of feeding devices provided by the embodiment of the present disclosure.

Animal activity data: this data reflects the activity level of feeding behavior of the animal during the second growth stage, including the eating frequency. The animal activity data can also be determined by interaction between communication devices. Specifically, the eating frequency of the animal is determined based on the interaction frequency between the communication transmitting device and the communication receiving device.

Animal weight data: this is an important indicator for assessing the growth condition of the animal. During the second growth stage, the weight of the animal will increase significantly. The weight data can be measured directly by a first weight estimation device integrated into the dividing device. The weight estimation device can be a weighing device, such as an electronic scale. Of course, it can be estimated by other indirect methods (e.g., by capturing the animal image through cameras and analyzing the image through the computer vision technique).

First environmental suitability data of the activity area: it is used to characterize the degree of environmental suitability of the animal, including environmental factors such as temperature, humidity, and air quality, which affect the growth and development of the animal. The environmental suitability data can be collected by specialized sensors. Specifically, sensors of temperature, humidity, carbon dioxide, and ammonia are integrated into the feeding device for feeding animals, which are used to collect environmental data of the activity area of the animals, thereby determining the first environmental suitability data.

Animal health condition data: it includes the disease incidence rate, vaccination condition, and medication records of animals in the second growth stage. Analyzing the health condition data of the animals can help to select more robust backup animals. Specifically, a disease assessment module is built on the cloud server. This module consists of two parts including treatment and immunization. The data of the two parts can be input by the management personnel of the breeding farm, or can be input by other modules, such as a medication management module. The medication management module records the medication condition and vaccination condition of each animal, and automatically transmits the recorded data to the disease assessment module, so that the disease assessment module derives the health condition data of the individual animal according to data of conditions of two aspects including the disease treatment and the immunization treatment for the individual animal.

Animal inbreeding condition data: it is an indicator for assessing the genetic diversity of animals and avoiding the risk of inbreeding. By recording and analyzing the strain and pedigree information of animals, it can avoid inbreeding during the selection and breeding process, thereby maintaining the genetic diversity and health of the population. Specifically, a lineage gene module is built on the cloud server. The module consists of two parts including the strain and the parental genes (represented by the parental identification) in the individual profile. The individual inbreeding index, i.e., the animal inbreeding condition data, can be assessed based on the two parts of the data.

In specific realization, the growth stage of the animal is divided into a first growth stage of feeding maternal milk and a second growth stage of eating feed. For the second growth stage, any one or more data of the animal eating data, animal activity data, animal weight data, first environmental suitability data of the activity area, animal health condition data, and animal inbreeding condition data are obtained. The backup animal meeting the preset selection requirement can be selected based on one or more of the above data of the second growth stage, or the backup animals meeting the preset selection requirement can also be selected by comprehensively considering the physiological data of the animal of two growth stages including the first growth stage and the second growth stage.

In the embodiment, by dividing the growth stages in detail and collecting multiple types of physiological data, it can more comprehensively and accurately assess the growth condition, health condition, and breeding potential of the animal. These data provide an important reference for subsequent selection and breeding decisions, which helps to improve the accuracy of selection and breeding.

Based on any one of the embodiments described above, the growth stages of the animal include a first growth stage and a second growth stage experienced sequentially; the animals are fed different foods during the first growth stage and the second growth stage; the physiological data of the first growth stage includes any one or more data of animal activity data, animal birth weight data, animal weaning weight data, second environmental suitability data of the activity area, animal mother eating data, animal mother health condition data, animal health condition data, and animal inbreeding condition data, wherein the animal activity data is determined based on the communication interaction data between the first communication transmitting device and the first communication receiving device, and the second environmental suitability data is configured to characterize a degree of environmental suitability of the animal mother.

It should be noted that animal birth weight data is a record of the birth weight of the animal, which is of significance in assessing the starting point of the growth and development. If the birth weight is too low, there is a certain probability that it is not suitable for a backup animal.

Animal weaning weight data: weaning is an important milestone in the animal growth process. The weaning weight data reflects the growth condition of the animal after leaving the maternal milk. Both the animal birth weight data and the animal weaning weight data play important roles in guiding the selection of backup animals.

Second environmental suitability data of the activity area: it is used to characterize the degree of environmental suitability of the animal mother in the first growth stage of the animal. Specifically, the first growth stage specifically refers to the farrowing stage of the animal. Four sensors of temperature, humidity, carbon dioxide, and ammonia are integrated into the feeding device that is configured for feeding the animal mother, which are used to collect environmental data of the activity area of the animal mother, thereby determining the second environmental suitability data. The second environmental suitability data can reflect the state of the animal mother just after giving birth. If the animal mother just after giving birth still has a high suitability degree to a harsh environment, it indicates that the animal mother has a good physical performance, and it has a larger probability of the animals born by the animal mother being the backup animal.

Animal mother eating data: eating condition of the animal mother also has a certain effect on the growth and development of the animal. The animal mother eating data is comprehensively determined by the feeding amount of the feeding device configured to feed the animal mother and the communication interaction data between the communication receiving device mounted on the feeding device and the communication transmitting device worn on the animal mother. Specifically, the first growth stage specifically refers to the farrowing stage of the animal. The animal mother eating data can reflect the state of the animal mother just after giving birth. If it is determined that the animal mother does not have an anorexia state just after giving birth based on the animal mother eating data, it indicates that the animal mother has a good physical performance, and it has a larger probability of the animal born by the animal mother being the backup animal.

Animal mother health condition data: the health condition of the animal mother directly affects the growth and development and health condition of the animal born by her. Therefore, the health condition of the animal mother gives a guide for the animal selection and breeding.

It should be understood that due to the uniqueness of the animal inbreeding condition data, if it needs to select the backup animal based on the physiological data of multiple growth stages, the animal inbreeding condition data can be obtained only once in the first growth stage, and the animal inbreeding condition data need not be obtained again in the subsequent growth stages. If it needs to select the backup animal based on the physiological data of one growth stage, the animal inbreeding condition data will be obtained once.

In the embodiment, by collecting and analyzing multiple physiological data in the first growth stage, it can more comprehensively and accurately assess the growth and development condition, health condition, and breeding potential of the animal. These data provide an important reference for subsequent selection and breeding decisions, which helps to improve the accuracy of selection and breeding.

Based on any one of the embodiments described above, the activity area includes a second weight estimation device, and the animal birth weight data and the animal weaning weight data are determined by the second weight estimation device.

It is noted that the second weight estimation device can be a removable integral machine. Specifically, if the selection and breeding animals are pigs, within 24 hours after the pig is born, the integral machine (on which the communication receiving device and the weighing device are integrated) is used to wear the two ears of the pig with the communication transmitting device in a special color and create a unique identification for the pig, wherein the odd number in the identification represents a boar, and the even number represents a sow. The pig profile is created in a cloud platform to form the lineage data, and at the same time, the birth weight measurement is completed and uploaded to the database. After weaning, the weight of pigs is measured again by using the integral machine, so as to obtain the animal weaning weight data. Of course, the animal weaning weight can also be measured by other devices, e.g., measured by a first weight estimation device integrated into the first feeding device.

In the embodiment, the second weight estimation device contained in the activity area provides an effective technical solution for determining the animal birth weight data and the weaning weight data. It can provide an accurate reference for subsequent selection and breeding decisions by accurately and reliably measuring and recording these data.

Based on any one of the embodiments described above, the animal mother wears a second communication transmitting device, and a second feeding device configured for feeding the animal mother is further mounted in the activity area, wherein the animal mother eating data is determined based on communication interaction data between the second communication transmitting device and a communication receiving device mounted on a second feeding device.

In the specific realization, the second feeding device consists of two parts including multiple sensors and the feeding mechanism, wherein the sensors mainly detect the material level and the eating desire of the animal, and the feeding mechanism mainly accomplishes the feeding on demand the calculation of the feeding amount. The accurate detection of the sensor minimizes the amount of waste, so that the feeding amount is infinitely close to the eating amount. This is because before feeding, the sensor will detect whether there is any feed left and how much feed is left, and then the feeding mechanism calculates the feeding amount based on the amount of feed left detected by the sensor so that the single feeding amount can be infinitely close to the eating amount. The feeding devices are further divided into the single feeding device for feeding a single animal and the group feeding device for feeding the plurality of animals. The daily feeding amount of the single feeding device is equal to the daily eating amount of an animal. For the group feeding device, based on the determination of the feeding amount, the daily eating amount of an animal is calculated based on the proportional relationship between the total daily eating duration of the animal individual and the total daily eating duration of the animal group. Whether the feeding device for the animal mother is a single feeding device or a group feeding device, the animal mother eating data can be calculated from the feeding amount and the eating duration, wherein the eating duration of the animal mother is determined based on the interaction duration between the communication transmitting device worn by the animal mother and the communication receiving device configured for feeding the animal mother. When the feeding device for feeding the animal mother is the single feeding device, the single feeding device is the second feeding device; and when the feeding device for feeding the animal mother is the group feeding device, the group feeding device is the second feeding device.

In the embodiment, through wearing the animal mother with the second communication transmitting device and mounting the second feeding device in its activity area, the real-time or timed recording and analyzing of the animal mother eating data can be realized.

Based on any one of the embodiments described above, an environmental detection device is integrated into each feeding device in the activity area, and the environmental detection device is configured to determine the environmental suitability data.

In one example, the breeding farm is divided into multiple activity areas; the animal and the animal mother live in the same activity area; and each activity area is provided with two types of feeding devices. One is a feeding device dedicated to feeding the animal, wherein this device can be a single feeding device or a group feeding device, and the other is a feeding device dedicated to feeding the animal mother, wherein this device can also be a single feeding device or a group feeding device. The environmental detection devices, such as four sensors of temperature, humidity, carbon dioxide, and ammonia, are integrated into both feeding devices in the activity area. Through the four sensors, the feeding device configured for feeding the animal collects environmental data of a smaller area where the animal is frequently active, thereby determining the first environmental suitability data characterizing the degree of environmental suitability of the animal; and also by the four sensors, the feeding device configured for feeding the animal mother collects environmental data of a smaller area where the animal mother is frequently active, thereby determining the second environmental suitability data characterizing the degree of environmental suitability of the animal mother.

In another example, the breeding farm is divided into multiple activity areas, and the animal and the animal mother live in different activity areas respectively. The activity area where the animal lives is provided with a feeding device A, and the activity area where the animal mother lives is provided with a feeding device B. The feeding device A and the feeding device B can be the same type of feeding device, i.e. the feeding device A and the feeding device B can both be single feeding devices or both be group feeding devices. Of course, the feeding device A and the feeding device B can be of different types of feeding devices. The environmental detection devices, such as four sensors of temperature, humidity, carbon dioxide, and ammonia, are integrated into both the feeding device A and the feeding device B. The feeding device A for feeding the animal collects the environmental data of the activity area where the animal lives by the four sensors, thereby determining the first environmental suitability data characterizing the degree of environmental suitability of the animal; and the feeding device B for feeding the animal mother collects the environmental data of the activity area where the animal mother lives by the four sensors, thereby determining the second environmental suitability data characterizing the degree of environmental suitability of the animal mother.

In another example, the breeding farm is divided into multiple activity areas. It is determined whether the animal and the animal mother live in the same activity area according to the growth stage of the animal. When the growth stage of the animal is the farrowing stage, the animal and the animal mother live in the same activity area. Two types of feeding devices are provided in this activity area, wherein one is the feeding device configured to feed the animal mother, and the feeding device can be the single feeding device or the group feeding device; and the other is the feeding device configured to feed the animal, and the feeding device can also be the single feeding device or the group feeding device. In another case, when the growth stage of the animal is the farrowing stage, the animal and the animal mother live in the same activity area. A feeding device provided with a drinking bowl is arranged in this activity area and mounted next to the farrowing bed, which facilitates the eating and drinking of the animal and animal mother. In another case, when the growth stage of the animal is the farrowing stage, whether or not the animal and its mother live in the same activity area, a drinking bowl is integrated next to the farrowing bed where the animal is located, and a feeding device for feeding the animal mother is arranged in the activity area where the animal mother is located. Since the animal at the farrowing stage mainly eats the maternal milk, it does not need to provide a feeding device for the animal during the farrowing period, and it only needs to mount the communication receiving device near the drinking bowl. When the animal comes close to the drinking bowl to drink water, the communication receiving device can be communicatively connected to the communication transmitting device on the animal, so as to determine the animal activity data. It should be noted that during the actual setup, the first feeding device for feeding the animal and the second feeding device for feeding the animal mother can be the same feeding device. The first feeding device and the second feeding device are only their identifications, and do not mean that they are two separate feeding devices. When the animal passes the farrowing stage and its growth stage is the nursery stage, the fattening stage, or other stages, the animal and the animal mother can live in different activity areas, at which time only one feeding device, dedicated to feeding the animal or the animal mother, needs to be arranged in each of the activity areas. No matter how many feeding devices are arranged in one activity area, the environmental detection devices, such as four sensors of temperature, humidity, carbon dioxide, and ammonia, are integrated into each feeding device. They are used to collect the environmental data, thus determining the environmental suitability data of the animal or the animal mother.

Additionally, during the farrowing stage of the animal, it usually needs to turn on a heating lamp to maintain the body temperature of the newborn animal. The environmental detection device (such as the temperature sensor) can be integrated into the heating lamp to monitor the temperature of the area covered by the heating lamp in real time. When the temperature reaches a certain standard, the heating lamp can be controlled to turn off, so as to conserve resources.

In the embodiment, the environmental detection device can monitor the environmental parameters around the feeding device in real time, such as temperature, humidity, and gas concentration (such as ammonia and carbon dioxide). This real-time monitoring helps to identify environmental problems in a timely manner and helps to determine the degree of environmental suitability of the animal.

Based on any one of the embodiments described above, the step S20 includes:
for each of the growth stages, determining a first breeding value of each of the growth stages according to each piece of the physiological data in the growth stages and a weight coefficient respectively corresponding to each piece of the physiological data;
calculating a second breeding value according to the first breeding value of each of the growth stages and a weight coefficient corresponding to each of the growth stages; and
determining the backup animal according to the size of the second breeding value of each animal.

As an example, when receiving the breeding instruction, the first physiological data of the first growth stage, the second physiological data of the second growth stage, and the third physiological data of the third growth stage for each animal are obtained, wherein the growth stages are divided based on ages of the animal. The first growth stage precedes the second growth stage, and the second growth stage precedes the third growth stage. The first growth stage includes the newborn stage of the animal, and the animal eats the maternal milk during the first growth stage; the second growth stage includes the nursery stage of the animal, and the animal is weaned in the second growth stage; and the third growth stage includes the growing-finishing stage of the animal, and the animal is released in the third growth stage. The first physiological data includes any one or more of the data of the animal mother eating data, the animal activity data, the animal birth weight data, the animal weaning weight data, the second environmental suitability data characterizing the degree of environmental suitability of the animal mother, the animal mother health condition data, the animal health condition data, and the animal inbreeding condition data. The second physiological data includes any one or more of the data of the animal eating data, the animal activity data, the animal weight data, the first environmental suitability data characterizing the degree of environmental suitability of the animal, and the animal health condition data. The third physiological data includes any one or more of the data of the animal eating data, the animal activity data, the animal weight data, the first environmental suitability data characterizing the degree of environmental suitability of the animal, and the animal health condition data. At least a part of the physiological data is determined based on communication interaction data between the first communication transmitting device and the first communication receiving device.

For each growth stage, each piece of physiological data in the growth stage is weighted and summed with the respective weight coefficient corresponding to each piece of physiological data to obtain a first breeding value for each growth stage.

The first breeding value of each growth stage is weighted and summed with the weight coefficient corresponding to each growth stage to obtain the second breeding value of each animal.

The backup animal is determined according to the second breeding value.

During the specific realization, the growth stage is divided into the farrowing stage (i.e., the newborn stage, the period from birth in the farrowing until being transferred to the nursery, and the farrowing stage generally lasts a few days), the nursery stage (the period from weaning to 70 days of age, and the nursery stage generally lasts 40 days), and the growing-finishing stage (the period after the nursery stage to the release date, and the growing-finishing stage generally lasts 6 months).

The following physiological data are collected during the farrowing stage, including the animal mother eating data, the animal activity data (since the animal eats the maternal milk during the farrowing stage, the drinking bowl is integrated into the feeding device for feeding the animal. The main function of the feeding device is to provide drinking water for the animal. The water is provided as the bait, and the feeding device is controlled to add the water. The animal is lured to drink water and close to the communication receiving device on the feeding device, so that the communication transmitting device and the communication receiving device on the animal are communicatively connected, and the animal activity data is determined based on the interaction data), the animal birth weight data, the animal weaning weight data, the second environmental suitability data characterizing the degree of environmental suitability of the animal mother, the animal mother health condition data, the animal health condition data, and the animal inbreeding condition data, wherein the weight coefficient corresponding to each physiological data can be set according to the actual selection and breeding goal. In this embodiment, the method of equal weight assignment is used to determine the weight coefficient of each physiological data. Taking the weight coefficient corresponding to each physiological data being 12.5% as an example, the first breeding value of the farrowing stage is a sum obtained by weighting summing eight different parts, wherein each part is a product of one factor (i.e., one physiological data collected during the farrowing stage) and its corresponding weight coefficient, e.g., the product of the animal mother eating data (the animal mother eating data can be a specific value or a group of values) and 12.5%.

The following physiological data are collected during the nursery stage, including the animal eating data, the animal activity data, the animal weight data, the first environmental suitability data of the degree of environmental suitability of the animal, and the animal health condition data, wherein the weight coefficient corresponding to each physiological data can be set according to the actual selection and breeding goal. In this embodiment, the method of equal weight assignment is used to determine the weight coefficient of each piece of physiological data. Taking the weight coefficient corresponding to each physiological data being 20% as an example, the first breeding value of the nursery stage is a sum obtained by weighting summing five different parts, wherein each part is a product of one factor (i.e., one physiological data collected during the nursery stage) and its corresponding weight coefficient, e.g., the product of the animal eating data (the animal eating data can be a specific value or a group of values) and 20%.

The following physiological data are collected during the growing-finishing stage, including the animal eating data, the animal activity data, the animal weight data, the first environmental suitability data of the degree of environmental suitability of the animal, and the animal health condition data, wherein the weight coefficient corresponding to each piece of physiological data can be set according to the actual selection and breeding goal. In this embodiment, the method of equal weight assignment is used to determine the weight coefficient of each piece of physiological data. Taking the weight coefficient corresponding to each piece of physiological data being 20% as an example, the first breeding value of the growing-finishing stage is a sum obtained by weighting summing five different parts, wherein each part is a product of one factor (i.e., one physiological data collected during the growing-finishing stage) and its corresponding weight coefficient, e.g., the product of the animal eating data (the animal eating data can be a specific value or a group of values) and 20%, and the first breeding value is obtained by adding the product of eight parts.

Later, the weight coefficient corresponding to each growth stage can be set according to the actual selection and breeding goal. In the embodiment, for example, the weight coefficient corresponding to the farrowing stage is 60%, the weight coefficient corresponding to the nursery stage is 10%, and the weight coefficient corresponding to the growing-finishing stage is 30%. The second breeding value of each animal is a sum obtained by weighting and summing three different parts, wherein one part is the product of the farrowing stage and its corresponding weight coefficient (60% as an example); another part is the product of the nursery stage and its corresponding weight coefficient (10% as an example); and the yet another part is the product of the growing-finishing stage and its corresponding weight coefficient (30% as an example). The second breeding value is obtained by adding the product of three parts.

The breeding selection algorithm module is integrated into the cloud server. The module is essentially a micro-intelligent decision body consisting of three parts: the algorithm model, the evaluation of the breeding value of the individual animal batch, and the assignment of identification of the backup animal. The calculation of the above breeding value is executed by the breeding algorithm module.

S is used to represent the breeding value, and the higher the S value is, the more suitable to be a backup animal. If there are N animals in the same batch of the backup animals to be selected, the second breeding values of the N animals are sorted from largest to smallest as follows:
S₁ > S₂ > S₃ > .........S_{N}.

It is to select the corresponding number of backup animals according to the order of sorting based on the number of required backup animals carried by the breeding instruction. For example, if the breeding instructions indicate that six backup animals are required, the identifications (such as identification numbers or identifications) of the first six animals are assigned to the dividing device, so that the dividing device divides these six animals to the breeding area according to the identifications.

In the embodiment, first, for each growth stage, the breeding value of each growth stage is determined according to each piece of physiological data in the stage and their respective corresponding weight coefficient. This means that the physiological data of each growth stage is assigned different importance (i.e., the weight coefficient), thereby obtaining a comprehensive breeding value. Next, the breeding value of the animal is calculated according to the breeding value of each growth stage and the weight coefficient corresponding to each growth stage. Finally, the backup animal meeting the preset selection requirement is determined according to the breeding value of each animal. Those animals with higher breeding values are considered as more potential backup animals. By comprehensively considering the physiological data of different growth stages and their importance and the effects of different growth stages on the selection and breeding of the backup animal, and by comprehensively assessing the breeding potential of each animal, the backup animals meeting the preset selection requirements can be more accurately determined, thereby improving the accuracy and efficiency of the selection and breeding.

Based on the method according to any above embodiments, the present disclosure further provides a structure schematic diagram of an electronic device as shown in FIG. 6. As shown in FIG. 6, at the hardware level, the electronic device includes a processor, an internal bus, a network interface, memory, and non-volatile storage, and of course, it can include other hardware required for the business. The processor reads the corresponding computer program from the non-volatile memory into memory, and then runs it, so as to realize the method according to any above embodiments.

Based on the method according to any above embodiments, the present disclosure further provides a computer storage medium, wherein the storage medium stores a computer program, and the computer program executes the method according to any one of the above embodiments when executed by the processor.

Based on the method according to any above embodiments, the present disclosure further provides a computer program product, wherein the computer program product includes one or more computer programs or instructions. The computer programs or instructions can be stored in the computer-readable storage medium or transmitted from one computer-readable storage medium to another computer-readable storage medium. The computer program realizes the method according to any above embodiments when executed by the processor.

In the embodiments provided by the present disclosure, it should be understood that the disclosed device and method can also be realized in other ways. The embodiments of the device described above are only illustrative, for example, the flow schematic diagrams and block schematic diagrams in the drawings show the architecture, functionality, and operation that may be realized by the device, method, and computer program product according to the plurality of embodiments of the present disclosure. At this point, each box in the flow schematic diagram or block schematic diagram may represent a module, program segment, or part of code, and the module, program segment, or part of the code includes one or more executable instructions for realizing specified logical functions. It should also be noted that in some embodiments as the replacement, the functions indicated in the boxes may also occur in a different order than those indicated in the drawings. For example, two consecutive blocks can actually be executed essentially in parallel, and they can sometimes be executed in reverse order, which depends on the functions involved. It is also noted that each block in the block diagram and/or flow schematic diagram, and combinations of boxes in the block diagrams and/or flow schematic diagrams, can be realized by a dedicated hardware-based system that executes the specified function or action or can be realized by a combination of the dedicated hardware and computer instructions.

Additionally, the various functional modules in various embodiments of the present disclosure can be integrated together to form a separate part, individual modules can exist alone, or two or more modules can be integrated to form a separate part.

If realized in the form of a software functional module and sold or used as an individual product, the function can be stored in a computer readable storage medium. Based on this understanding, the technical solution of the present disclosure in essential, the part contributing to the prior art, or the part of the technical solutions can be embodied in the form of the software product. The computer software product is stored in a storage medium, including several instructions to cause a computer device (which may be a personal computer, a server, or a network device, etc.) to perform all or part of the steps of the method described in each embodiment of the present disclosure. The foregoing storage medium includes U disk, portable hard disk, ROM (read only-memory), RAM (random access memory), disk, disc, or other media that can store the program code.

The foregoing are merely embodiments of the present disclosure and are not used to limit the scope of the protection of the present disclosure. For those skilled in the art, the present disclosure may have various changes and variations. Any modifications, equivalent replacements, improvements, etc., made within the spirit and principles of the present disclosure, shall be included within the scope of protection of the present disclosure. It should be noted that similar symbols and letters denote similar items in the following drawings, so that once an item is defined in a drawing, no further definition or explanation is required in the subsequent drawings.

The above-mentioned are only specific embodiments of the present disclosure, but the scope of protection of the present disclosure is not limited thereto. Any person skilled in the art familiar with the technical field, who can easily think of variations or substitutions within the scope of the technology disclosed in the present disclosure, shall be covered by the scope of protection of the present disclosure. Therefore, the scope of protection of the present disclosure shall be governed by the scope of protection of the claims.

It should be noted that in the text, relational terms such as "first", "second", etc., in the description of the present disclosure are only used to distinguish one entity or operation from another entity or operation, and do not necessarily require or imply the existence of any such actual relationship or order between these entities or operations. Furthermore, the terms "include", "comprise", or any other variant thereof are intended to cover a non-exclusive inclusion so that a process, method, item, or device comprising a set of components includes not only those components, but also another component that is not explicitly listed, or component are inherent to this process, method, item, or device. Without further limitation, the fact that a component is defined by the phrase "including a ..." does not exclude the existence of another identical component in the process, method, item, or device including the component.

## Claims

1. An animal selection and breeding method, wherein each animal wears a first communication transmitting device, and a dividing device and a first feeding device for feeding animals are mounted in an activity area of the animals, wherein a first communication receiving device is mounted on the first feeding device, and the first communication receiving device is communicatively connected to the first communication transmitting device in a preset range; and the method comprises:
obtaining physiological data of each of the animals at one or more growth stages when receiving a breeding instruction, wherein the growth stages are divided based on ages of the animals, and at least a part of the physiological data is determined based on communication interaction data between the first communication transmitting device and the first communication receiving device;
determining backup animals meeting preset selection requirements from the multiple animals based on the physiological data; and
sending a dividing instruction with an identification of the backup animals to the dividing device, wherein the dividing instruction is configured to instruct the dividing device to divide the backup animals to a breeding area.

2. The method according to claim 1, wherein the growth stages of the animal comprise a first growth stage and a second growth stage experienced sequentially; the animals are fed different foods during the first growth stage and the second growth stage; the physiological data of the second growth stage comprises any one or more types of data of animal eating data, animal activity data, animal weight data, first environmental suitability data of the activity area, animal health condition data, and animal inbreeding condition data, wherein the animal eating data and the animal activity data are determined based on the communication interaction data between the first communication transmitting device and the first communication receiving device; the animal weight data is determined based on a first weight estimation device integrated into the first feeding device; and the first environmental suitability data is configured to characterize a degree of environmental suitability of the animals.

3. The method according to claim 1, wherein the growth stages of the animal comprise a first growth stage and a second growth stage experienced sequentially; the animals are fed different foods during the first growth stage and the second growth stage; the physiological data of the first growth stage comprises any one or more types of data of animal activity data, animal birth weight data, animal weaning weight data, second environmental suitability data of the activity area, animal mother eating data, animal mother health condition data, animal health condition data, and animal inbreeding condition data, wherein the animal activity data is determined based on the communication interaction data between the first communication transmitting device and the first communication receiving device, and the second environmental suitability data is configured to characterize a degree of environmental suitability of the animal mother.

4. The method according to claim 3, wherein the activity area comprises a second weight estimation device, and the animal birth weight data and the animal weaning weight data are determined by the second weight estimation device.

5. The method according to claim 3, wherein the animal mother wears a second communication transmitting device, a second feeding device configured for feeding the animal mother is further mounted in the activity area, and the animal mother eating data is determined based on communication interaction data between the second communication transmitting device and a communication receiving device mounted on a second feeding device.

6. The method according to any one of claims 1-5, wherein an environmental detection device is integrated into the feeding device of the activity area, and the environmental detection device is configured to determine the environmental suitability data.

7. The method according to claim 1, wherein the step of determining backup animals meeting preset selection requirements from the multiple animals based on the physiological data comprises:
determining, for each of the growth stages, a first breeding value of each of the growth stages according to each piece of the physiological data in the growth stages and a weight coefficient respectively corresponding to each piece of the physiological data;
calculating a second breeding value according to the first breeding value of each of the growth stages and a weight coefficient corresponding to each of the growth stages; and
determining the backup animals according to the second breeding value of each of the animals.

8. An electronic device, wherein the electronic device comprises:
a processor,
configured to store a memory of processor-executable instructions, wherein
the processor realizes the method according to any one of claims 1-7 when the processor invokes the executable instructions.

9. A computer-readable storage medium, storing computer instructions, wherein the computer program instructions execute steps of the method according to any one of claims 1-7 when executed by the processor.

10. A computer program product, wherein the computer program product comprises computer programs, and the method according to any one of claims 1-7 is realized when the computer programs are executed by the processor.
